Europäisches Patentamt

European Patent Office

Office européen des brevets

(11) Numéro de publication: **0 292 351**
**A1**

# (12) DEMANDE DE BREVET EUROPEEN

(21) Numéro de dépôt: 88401101.6

(51) Int. Cl.⁴: **G 06 F 15/74**

(22) Date de dépôt: 05.05.88

(30) Priorité: 12.05.87 FR 8706657

(43) Date de publication de la demande:
23.11.88 Bulletin 88/47

(84) Etats contractants désignés: **DE GB IT**

(71) Demandeur: **COMMISSARIAT A L'ENERGIE ATOMIQUE**
**31/33, rue de la Fédération**
**F-75015 Paris (FR)**

(72) Inventeur: **Invernizzi, Michel**
**13, rue des Molières**
**F-91400 Orsay (FR)**

**Minault, Sophie**
**14, rue du Parc**
**F-91440 Bures Sur Yvette (FR)**

(74) Mandataire: **Mongrédien, André et al**
**c/o BREVATOME 25, rue de Ponthieu**
**F-75008 Paris (FR)**

(54) **Système de traitement de signal.**

(57) Le système comprend un moyen de prétraitement (2) recevant un ou plusieurs signaux monodimensionnels S et délivrant une suite de symboles dits terminaux $S_T$ constituent un codage des signaux reçus, et un moyen de traitement (4) recevant ladite suite et délivrant une autre suite de symboles dits sommés $S_S$ formant une représentation condensée des signaux S, ladite suite de symbole sommet permettant d'interpréter l'information représentée par les signaux S.

Le moyen de traitement (4) comprend un registre (12) pour recevoir la suite de symboles terminaux $S_T$, une mémoire (14) contenant un ensemble de règles de récriture mémorisé dans des cellules de mémorisation organisées en un arbre logique, et un moyen de codage (16) pour comparer les symboles en tête dudit registre avec les règles de récriture rangées dans un ordre déterminé, cet ordre correspondant au choix prioritaire du chemin le plus long en chaque noeud de l'arbre logique.

Application à la reconnaissance de la parole, au traitement de signaux de type électrocardiogramme et autre.

FIG. 1

**Description**

## SYSTEME DE TRAITEMENT DE SIGNAL

La présente invention a pour objet un système de traitement de signal du type de ceux qui analysent un signal reçu pour identifier dans ce signal des formes caractéristiques. La représentation de ce signal par une suite de formes caractéristiques permet ensuite une interprétation aisée de ce signal, cette interprétation pouvant être réalisée soit par une personne soit par une machine automatique.

L'invention s'applique dans tous les domaines où il peut être nécessaire de traiter un signal pour le rendre plus facilement interprétable. L'invention s'applique notamment en reconnaissance de la parole pour la reconnaissance automatique d'un signal de parole et en médecine pour l'interprétation de signaux tels que électrocardiogrammes, électroencéphalogrammes ou autres.

En reconnaissance de la parole, plusieurs systèmes de traitement existent. Des exemples, de tels systèmes de traitement sont décrits notamment dans l'article "The HEARSAY II Speech Undestanding System : Integrating Knowledge to Resolve Uncertainly" de L.D. ERMAN et al. paru dans Computing Surveys, vol.12, n°2, Juin 1980 et dans l'article "Un système expert pour la reconnaissance de la parole" de M. GILLOUX et al. présenté au Colloque International d'Intelligence Artificielle, Marseille, oct.1984.

On connaît également un système de traitement de signal pour l'interprétation d'un signal en géologie. Un tel système est décrit par exemple dans l'article "DIPMETER ADVISOR : on Système Expert en Interprétation Géologique" de J. luu présenté aux Journées d'Etudes Systèmes Experts, AFCET ADI Avignon, 2-4 mai 1984.

Les systèmes de traitement de signal décrits dans les articles cités ci- dessus appartiennent à la famille dite des systèmes experts. La structure d'un tel système est un peu particulière et mérite d'être rappelée brièvement. Pour une présentation plus complète, on pourra se reporter à l'ouvrage "Systèmes experts, méthodes et outils" de J.N. Chatain, ed. Eyrolles, oct.1986, ainsi qu'aux documents indiqués dans la bibliographie de cet ouvrage.

Un système expert en traitement de signal comprend trois parties principales :

- une base de faits, constitués d'une mémoire, contenant initialement une représentation du signal à traiter,

- une base de connaissances qui contient l'ensemble de la connaissance spécialisée introduite par un expert humain du domaine technique ; cette connaissance est représentée sous la forme d'un ensemble de règles dites de production et est codée sous forme numérique dans une mémoire, et

- un moteur d'inférence qui constitue le moyen de traitement ; ce moteur d'inférence met en oeuvre les éléments de la base de connaissance pour construire des raisonnements. Il exécute ainsi des inférences, ou déductions, qui permettent de modifier les éléments de la base de faits pour obtenir finalement le signal traité voulu.

Un système expert en traitement du signal comprend aussi généralement une interface interactive pour permettre à un opérateur de demander la visualisation du signal contenu dans la base de faits, de connaître le raisonnement suivi par le moteur d'inférence, de modifier les règles de production de la base de connaissances et de manière générale pour consulter ou modifier tout élément du système expert.

De manière générale, la base de connaissances est constituée d'une table contenant des règles de production indépendantes. Une règle de production est une expression de la forme :

Si prémisse alors(conclusion)

La partie gauche de la règle (prémisse) exprime les conditions d'applicabilité de la règle. Elle peut contenir une conjonction de propositions logiques, de faits ou de relations. La partie droite (conclusion) représente la conclusion qui peut être une action à effectuer ou une assertion ajouter dans la base de faits.

L'un des inconvénients majeurs des systèmes de traitement de signal tels que les systèmes experts pour la reconnaissance de la parole ou l'interprétation géologique cités plus haut est leur lenteur.

Celle-ci est inhérente au mode de traitement mis en oeuvre par le moteur d'inférence. En effet, les règles de la base de connaissances sont essayées l'une après l'autre sur le signal contenu dans la base de faits, sans qu'il soit tiré profit des éventuelles redondances dans la base de connaissances. Ceci a notamment pour conséquence que de nombreuses règles sont testées inutilement.

Un gain de vitesse de traitement a été obtenu en procédant, comme décrit dans l'article "Rete : a fast Algorithm for the Many Pattern/many Object Pattern Match Problem" de Charles L. FORGY paru dans Artificial Intelligence, vol.19, 1982, pp.17-37, à une compilation des règles alors qu'elles étaient auparavant interprétées.

Cependant, cette procédure connue ne peut être appliquée directement à un traitement de signal.

Un autre inconvénient important des systèmes de traitement de signal évoqués ci-dessus est que le signal à interpréter doit d'abord subir un long prétraitement (transformation de Fourier, filtrage numérique) pour en dégager des paramètres et caractéristiques qui, par avance, sont jugés pertinents et que ce n'est que sur l'information issue de ce long prétraitement que le système expert peut travailler.

L'invention a notamment pour but de simplifier le traitement du signal en permettant de travailler directement sur ce dernier sans qu'il soit nécessaire de faire un prétraitement long et élaboré.

L'invention a également pour but d'augmenter la vitesse du traitement du signal, en organisant la base de connaissances de manière supprimer les redondances dans la recherche des règles applicables.

L'invention consiste dans un système de traitement de signal qui réalise successivement deux traitements. Dans une première étape un moyen de prétraitement permet de remplacer le signal à traiter, qui est par exemple un signal représentant l'évolution de l'amplitude d'une grandeur physique en fonction du temps, par une suite de symboles dits symboles terminaux (en référence à la terminologie employée en théorie de la compilation). Ce prétraitement permet de décrire le signal à traiter à l'aide d'un ensemble de symboles prédéterminés, ce qui en facilite son analyse.

Dans une seconde étape, la suite de symboles terminaux est remplacée progressivement par une autre suite de symboles, dits symboles sommets, qui correspondent chacun à une forme particulière d'un élément du signal.

Le traitement mis en oeuvre dans cette seconde étape se caractérise d'une part en ce qu'il utilise un ensemble de règles de production particulières, dites règles de récriture, pour relier les symboles terminaux et les symboles sommets et, d'autre part, en ce que ces règles sonnt stockées dans une mémoire selon une structure en arbre.

L'organisation des règles en arbre permet, en combinaison avec les techniques classiques de parcours d'un arbre, de trouver beaucoup plus rapidement que dans les systèmes de traitement connus la règle applicable à une suite de symboles.

De manière précise, l'invention a pour objet un système de traitement de signal comprenant:

- un moyen de prétraitement muni d'une entrée pour recevoir un ou plusieurs signaux monodimensionnels et d'une sortie, ledit moyen de prétraitement comprenant une mémoire contenant un ensemble E de symboles $S_T$ dits terminaux, chaque symbole étant un code associé à une forme simple prédéterminée du signal, et un moyen de codage pour analyser ledit signal S et délivrer sur ladite sortie dudit moyen de prétraitement une suite de symboles terminaux constituant un codage dudit signal S,

- un moyen de traitement muni d'une entrée pour recevoir ladite suite de symboles terminaux, et pour délivrer une suites de symboles dits sommets correspondante, ladite suite de sommets formant une représentation condensée dudit signal S, où chaque symbole sommet représente une forme complexe prédéterminée de signal égale à une suite de plusieurs formes simples, ledit moyen de traitement comprenant :

    a) un registre de type premier entré-premier sortie pour recevoir les symboles terminaux délivrés par le moyen de prétraitement,

    b) une mémoire contenant un ensemble de règles de récriture, chaque règle R étant de type MG $\rightarrow$ MD, où les éléments MG et MD sont des séquences de symboles comprenant des symboles sommets et des symboles terminaux, ledit ensemble de règles de récriture étant mémorisé daans des cellules de mémorisation organisées en un arbre logique comprenant : une cellule de mémorisation appelée racine, un ensemble de cellules de mémorisation appelées noeuds finaux et un ensemble de cellules de mémorisation appelées noeuds intermédiaires, ladite racine comprenant une zone pour contenir des pointeurs réalisant un chaînage avec des noeuds intermédiaires, et chaque noeud intermédiaire comprenant une zone pour contenir un symbole et une zone pour contenir des pointeurs réalisant un chaînage avec un noeud final ou au moins un autre noeud intermédiaire, lesdits chaînages formant des chemins entre la racine et chaque noeud final, chaque chemin correspondant à une séquence de symboles consituant l'élément MG d'une règle R et le noeud final situé à l'extrémité dudit chemin comprenant une zone pour contenir l'élément MD de ladite règle R, et

    c) un second moyen de codage pour comparer les symboles placés en tête dudit registre avec les séquences MG de chaque règle et remplacer une suite desdits symboles, identique à une séquence MG d'une règle de récriture par la séquence MD de ladite règle, ledit second moyen de codage délivrant sur ladite sortie dudit moyen de traitement chaque symbole sommetplacé en tête dudit registre et répétant l'opération de remplacement des symboles en tête tant que le registre n'est pas vide, ledit second moyen de codage étant conçu pour comparer les symboles en tête dudit registre avec les éléments MG des règles rangées dans un ordre déterminé, cet ordre correspondant au parcours dudit arbre logique en choisissant à chaque noeud intermédiaire le chemin le plus long d'abord.

A chaque séquence de symboles formant un élément MG d'une règle est donc associé un chemin entre le noeud initial et un noeud final, ce chemin comportant un ensemble de noeuds intermédiaires mémorisant chacun un symbole de ladite séquence.

Dans la séquence de symboles lue de gauche à droite, le symbole de rang i peut être mémorisé dans le noeud intermédiaire de rang i, compté à partir du noeud initial. Les symboles sont alors stockés dans l'ordre du noeud intial vers le noeud final.

Toutefois, de manière préférée, le symbole de rang i de la séquence est stocké dans le noeud intermédiaire du rang i, compté à partir du noeud final. Les symboles sont alors stockés dans l'ordre inverse du noeud initial vers le noeud final. Cette structure présente dans de nombreux cas, l'avantage de permettre un traitement de signal plus rapide.

De manière préférée, dans cette variante, chaque noeud final contenant une séquence de symbole S(1), ... S(m), $1 \leqq m$, formant un élément MD d'une règle R, qui est égale à une séquence de symboles terminant un élément $MG_1$, contient en outre un pointeur réalisant un chaînage avec le noeud intermédiaire, du chemin correspondant audit élément $MG_1$, contenant ledit symbole S(1).

Ceci procure un gain de temps par rapport à un traitement qui consisterait à reprendre systématiquement à son début l'exploration de l'arbre après l'application d'une règle.

Selon un mode de réalisation particulier, au moins certains symboles terminaux et/ou certains symboles sommet comportent des attributs. Ces attributs permettent de paramétrer la forme associée au symbole, par exemple en précisant sa largeur, sa hauteur ou autre.

La règle applicable dépend en général de la valeur des attributs. De manière préférée, lorsqu'un élément MG d'une règle comporte au moins un symbole avec attributs, tous les tests portant sur la nature des symboles sont faits avant les tests portant sur les valeurs des attributs. Les tests sur les attributs, qui sont plus longs que les tests sur les symboles, ne sont ainsi réalisés que si tous les tests sur les symboles sont positifs.

Les caractéristiques et avantages de l'invention ressortiront mieux de la description qui va suivre, donnée titre illustratif mais non limitatif, en référence aux dessins annexés, sur lesquels :

- la figure 1 représente schématiquement le système de traitement de signal de l'invention,

- la figure 2 illustre schématiquement un signal à traiter et la suite de symboles terminaux correspondants produite par le moyen de prétraitement du système de l'invention,

- la figure 3 illustre la structure en arbre de la base de connaissances constituée des règles R1-R6,

- la figure 4 montre schématiquement l'organisation de la mémoire correspondant à la structure en arbre de la figure 3,

- la figure 5 illustre le procédé mis en oeuvre par le moyen de traitement du système de l'invention, et

- la figure 6 illustre un deuxième mode de représentation en arbre de la base de connaissances constituée des règles R1-R6.

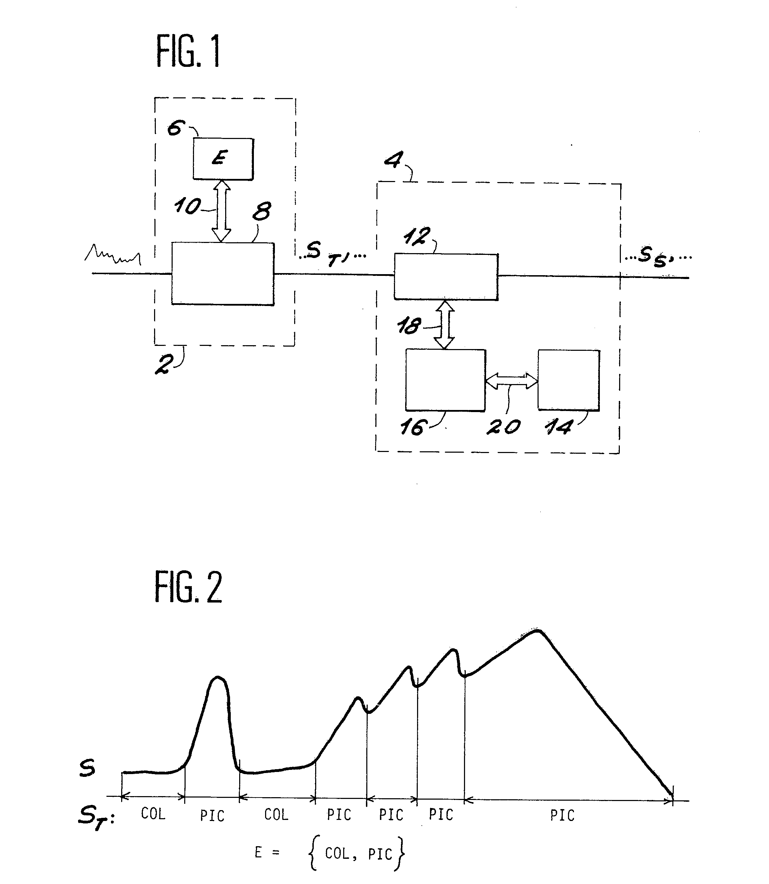

On a représenté schématiquement sur la figure 1 un système de traitement de signal selon l'invention.

Le système comporte un moyen de prétraitement 2 et un moyen de traitement 4. Il reçoit un signal monodimensionnel numérisé qui représente par exemple l'amplitude d'une grandeur physique en fonction du temps, tel qu'un signal de parole reçu d'un microphone ou un électrocardiogramme.

Le moyen de prétraitement 2 comprend une mémoire 6 contenant un ensemble E de symboles $S_T$, dits symboles terminaux, chaque symbole étant associé à une forme particulière, par exemple, à un échantillon de signal.

Le moyen de prétraitement 2 comprend également un premier moyen de codage 8 et une voie 10 pour lire le contenu de la mémoire 6. Le premier moyen de codage 8 reçoit le signal à traiter et délivre une suite de symboles terminaux représentative dudit signal.

Les symboles terminaux peuvent par exemple être associés à des formes telles qu'un pic, une plage constante ou autre.

A titre d'exemple, on a représenté schématiquement sur la figure 2 un signal S reçu par un moyen de prétraitement dont la mémoire contient un ensemble E de symboles terminaux réduit aux seuls éléments "col" et "pic", où "col" indique un signal sensiblement constant sur une longueur supérieure à un seuil et "pic" indique un signal passant par un maximum. La suite de symboles terminaux correspondant au signal S est représentée sous celui-ci.

Pour que la représentation du signal S par une suite de symboles terminaux soit fidèle, on peut adjoindre à chaque élément de l'ensemble E un ou plusieurs attributs. Par exemple, un paramètre "largeur" peut être associé au symbole "col", et des paramètres "largeur" et "altitude" peuvent être associés au symbole "pic".

Les symboles terminaux sont associés à des formes simples du signal qui ne représentent pas elles-mêmes une caractéristique permettant de reconnaître ou d'interpréter le signal. Il faut auparavant regrouper de manière successive des séquences de symboles terminaux pour identifier des formes complexes du signal, qui constituent une information sur le contenu du signal.

L'ensemble de ces regroupements est réalisé par le moyen de traitement 4 du système représenté sur la figure 1.

Ce moyen de traitement 4 comprend un registre 12 de type premier entré-premier sorti pour mémoriser les symboles terminaux $S_T$ reçus du moyen de prétraitement 2 dans l'ordre de leur arrivée.

Il comprend également une mémoire 14 contenant un ensemble R de règles de récriture qui permettent de transformer la suite de symboles terminaux reçue en une suite de symboles sommets associés à des formes complexes du signal.

La transformation est réalisée par un second moyen de codage 16 reliée au registre 12 par une voie 18 et à la mémoire 14 par une voie 20.

Le nombre de règles contenues dans la mémoire 14 dépend de l'application considérée, mais elle est en général de l'ordre de quelques dizaines à une centaine de règles.

Pour reprendre l'exemple de la figure 2, on peut considérer un ensemble de six règles permettant de définir trois formes complexes. Ces formes complexes sont désignées par les symboles "chicot", "cuesta" et "pic-large" :

R1 : pic (largeur < L1)→pic-étroit
R2 : pic (largeur > L1)→pic-large
R3 : col, pic-étroit→début de cuesta
R4 : col, pic-étroit, col→chicot
R5 : début de cuesta, pic-étroit→début de cuesta
R6 : début de cuesta, pic-large→cuesta, pic-large

Ces règles définissent un "chicot" comme un "pic-étroit" entouré de deux "cols" ; une "cuesta" comme une suite de "pic-étroits" et un "pic-large" comme un "pic" dont la largeur est supérieure à un seuil L1.

On note que ces règles définissent des symboles sommets mais aussi de nouveaux symboles terminaux ("début de cuesta", "pic-étroit") qui sont associés à des formes intermédiaires entre les formes simples (cf. figure 2) et les formes complexes associées aux symboles sommets.

L'invention repose d'une part sur l'utilisation de règles de récriture pour le traitement du signal et d'autre part sur une structure particulière des règles dans la mémoire 14 pour supprimer les tests redondants.

De manière précise, les règles sont rangées selon une structure en arbre comprenant un noeud initial, des noeuds intermédiaires et des noeuds finaux. Les éléments MG et MD d'une règle de type MG → MD sont représentés respectivement par un chemin constitué d'une suite de noeuds intermédiaires reliant le noeud initial et un noeud final, et par ledit noeud final.

Conformément à l'invention, cet arbre est parcouru dans un ordre correspondant au test des règles dont l'élément MG est le plus long. Il s'agit là d'un souci d'efficacité maximale.

En effet, supposons que l'on applique la première règle possible à une suite de symboles contenus dans le registre. Dans ce cas, si cette suite est col, pic-étroit, col, ... la règle R3 s'applique puisque les deux premiers symboles de la suite sont col et pic-étroit. Au contraire, si on applique la règle la plus longue d'abord, c'est R4 qui s'applique et on traite en une fois un plus grand nombre de symboles.

Pour tester si une règle est applicable, on compare chacun des symboles de l'élément MG de la règle avec une suite de symboles du registre. la comparaison peut être faite dans l'ordre des symboles de l'élément MG, c'est-à-dire du symbole le plus à gauche vers le symbole le plus à droite, ou dans l'ordre inverse.

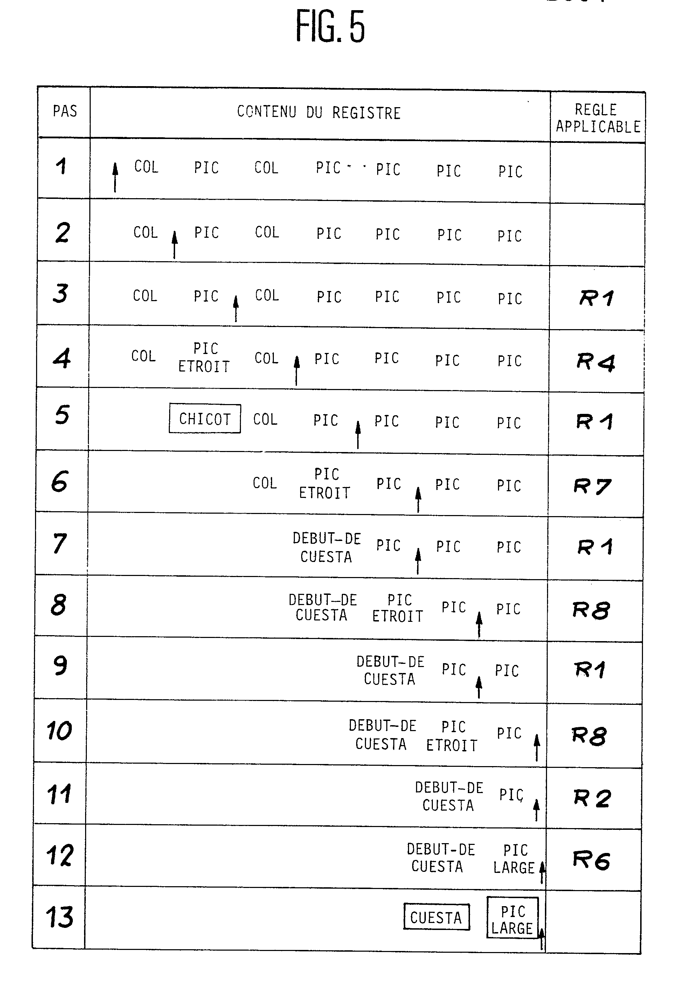

Un arbre correspondant au premier mode de comparaison est représenté sur la figure 5, et sera décrit ultérieurement.

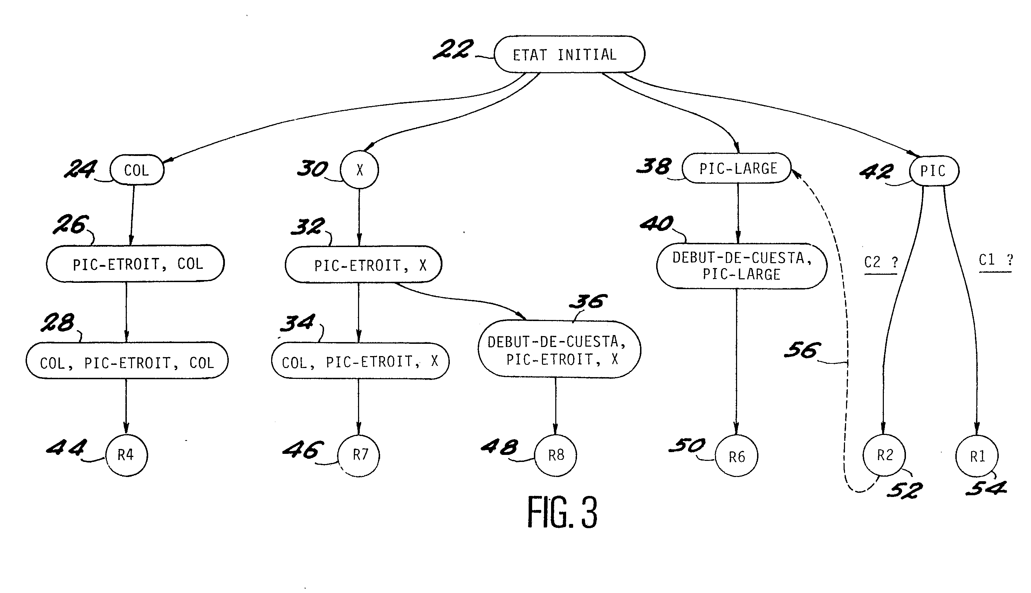

De manière préférée, le moyen de traitement fonctionne selon le second mode de comparaison. Un arbre représentatif de l'organisation de la mémoire pour la mise en oeuvre de ce mode de comparaison est représenté sur la figure 3.

Cet arbre comporte un noeud initial 22, un ensemble de noeuds intermédiaires 24-42 et un ensemble de noeuds finaux 44-54. Le noeud initial 22 représente un état initial et constitue uniquement un moyen d'entrée de l'arbre.

Les noeuds intermédiaires sont disposés entre le noeud initial et les noeuds finaux et constituent des chemins qui correspondent chacun à une règle. Par exemple, la suite des noeuds 24, 26, 28 représente le test d'applicabilité de la règle R4.

Le noeud 24 représente la comparaison entre un symbole du registre 12 et le dernier symbole de l'élément MG de la règle R4. Le noeud 26 correspond au test : "le symbole précédent "col" dans le registre est-il "pic-étroit"?" De même, le noeud 28 correspond au test : "le symbole précédant les symboles "col" et "pic-étroit" dans le registre est-il le symbole "col" ?". Si les trois tests correspondant aux noeuds 24, 26 et 28 sont positifs, la règle R4 s'applique.

L'arbre représenté sur la figure 3 correspond à un second moyen de codage fonctionnant comme un analyseur syntaxique à pile. Cette pile est constituée par un pointeur qui désigne un symbole de rang déterminé du registre du moyen de traitement.

Au début, le second moyen de codage recherche si une règle est applicable au premier symbole du registre. Le pointeur désigne ce symbole (la pile contient uniquement ce symbole). Si aucune règle n'est applicable, le pointeur est déplacé vers le second symbole du registre (la pile contient les deux premiers symboles du registre). L'arbre est parcouru nouveau pour recherche si une règle est applicable.

Pour que ce procédé de traitement soit compatible avec le critère consistant à tester d'abord les règles dont l'élément gauche MG est le plus long, il est connu de compléter l'élément gauche MG1 d'une règle par un ou plusieurs symboles déterminés "X" lorsque cet élément gauche MG1 est aussi le début d'un élément gauche MG2 d'une autre règle. Une telle technique est classique dans les analyseurs syntaxiques à pile et est décrite notamment dans l'ouvrage "Compiler Construction for Digital Computer" de David GRIES, chap.7, pp.155-169 édité en 1971 par Wiley International Edition. Dans cet ouvrage, le symbole "X" est noté ANY.

Ainsi, pour que la règle R3 ne soit testée qu'après la règle R4, la règle R3 est remplacée par une règle R7 : col, pic-étroit, X→ début de cuesta, X.

Cette modification de la règle R3 entraîne une modification de toutes les règles dont l'élément gauche se termine par le même symbole que l'élément gauche de la règle R3. C'est le cas pour la règle R5 qui est remplacée par une règle R8 : début de cuesta, pic-étroit, X→ début de cuesta, X

Ceci résulte du fait que les règles R3 et R5 font partie du même sous-arbre commençant par un test sur le symbole pic-étroit. Si le sous-arbre est complété par l'adjonction d'un nouveau test sur un symbole "X", ce test occupant la racine de l'arbre, le nouveau test s'applique à toutes les règles définies dans le sous-arbre.

L'arbre étant ainsi complété, comme représenté sur la figure 3, le test des règles dans l'ordre désiré est réalisé en parcourant l'arbre de gauche à droite et en profondeur d'abord.

Après chaque récriture, c'est-à-dire lorsqu'on a trouvé la règle applicable et que l'on a remplacé, dans le registre, la suite de symboles égale à l'élément gauche MG de la règle par la suite de symboles égale à l'élément droite de la règle, on revient en général à l'état initial.

Cependant, il peut arriver que la nouvelle suite de symboles constitue elle-même un membre gauche ou une partie de membre gauche d'une autre règle. Dans ce cas, il est intéressant de tester directement si cette règle est applicable sans perdre de temps à tester les règles plus longues, donc plus prioritaires, qui ne sont pas applicables.

Ainsi, l'application de la règle R2 remplace un symbole "pic" par un symbole "pic-large" qui est aussi le dernier symbole du membre gauche de la règle R6. Il est donc avantageux de ne pas reprendre le traitement partir de l'état initial après l'application de la règle R2 (noeud 52), mais de se brancher directement sur le noeud 38. On fait ainsi l'économie des tests inutiles correspondant aux noeuds 24, 30 et 32.

Les arcs notés C1? et C2? entre le noeud 42 et les noeuds 52 et 54 représentent des tests sur les valeurs des attributs des symboles. Le test C1? vérifie si la largeur du symbole "pic" est inférieure au seuil L1, ce qui conditionne l'applicabilité de la règle R1. De même, le test C2? vérifie si la largeur du symbole "pic" est supérieure au seuil L1, ce qui conditionne l'applicabilité de la règle R2.

De manière générale, lorsque l'application d'une règle dépend de la présence de plusieurs symboles et de la valeur des attributs de certains de ces symboles, on teste d'abord l'ensemble des symboles et ensuite l'ensemble des attributs. Ceci permet un gain de temps car les tests sur les attributs, qui sont coûteux en temps de calcul, sont ainsi effectués moins souvent que si l'attribut de chaque symbole était testé en même temps que ledit symbole.

L'arbre représenté sur la figure 3 est matérialisé dans la mémoire 14 du moyen de traitement dans des cellules de mémorisation comprenant des zones pour mémoriser des symboles et des zones pour mémoriser des pointeurs qui représentent les chaînages entre les nœuds de l'arbre.

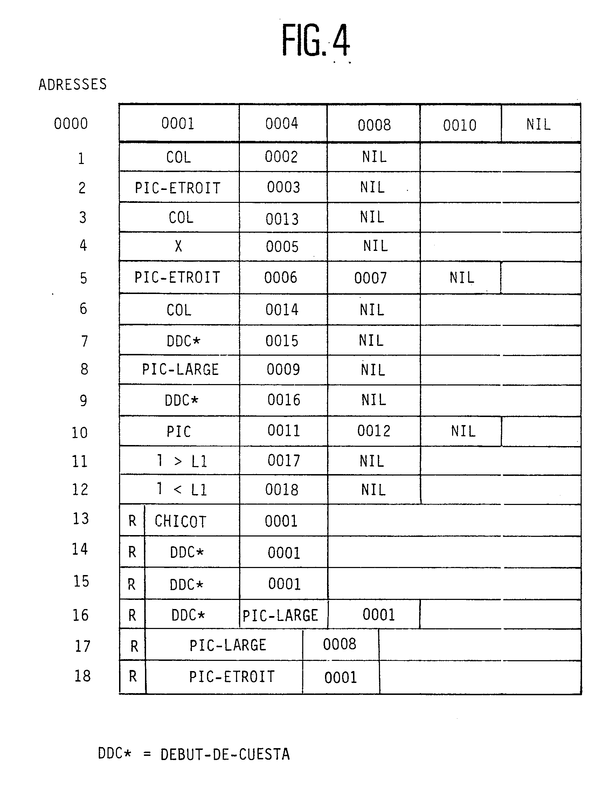

Une organisation classique de la mémoire est représentée sur la figure 4. Dans celle-ci chaque cellule de mémorisation possède une adresse et contient l'ensemble des informations associées à un noeud.

La cellule d'adresse 0000 mémorise le noeud initial 22. Elle comporte une suite de zones contenant chacune un pointeur pour désigner les noeuds 24, 30, 38 et 42.

Les cellules d'adresses 0001 à 0010 correspondent aux noeuds intermédiaires 24-42. Elles comportent chacune une zone contenant le symbole à tester, et au moins une zone contenant chacune un pointeur pour indiquer le sens de parcours de l'arbre.

Les cellules d'adresses 0011 et 0012 concernent les tests $\underline{C2?}$ et $\underline{C1?}$ de l'arbre.

Les cellules d'adresses 0013 à 0018 mémorisent chacune un noeud final. Elles comprennent éventuellement une zone pour contenir un code "R" indiquant qu'il s'agit d'un noeud final, une suite de zones énonçant la suite des symboles constituant le membre droit MD de la règle, et une zone pour mémoriser un pointeur indiquant le branchement à effectuer.

De manière générale, après l'application d'une règle, le traitement reprend au noeud initial d'adresse 0001. Cependant, comme indiqué par l'arc 56 sur la figure 3, on peut avantageusement effectuer un branchement différent dans certains cas. Cet arc 56 se traduit par une valeur 0008 dans la zone de pointeur de la cellule de mémorisation d'adresse 17.

Pour les cellules de mémorisation d'adresses 0013 à 0018, le branchement est inconditionnel et unique ; il suffit donc d'un seul pointeur. En revanche, pour les cellules d'adresses 0000 à 00012, les branchements dépendent du résultat des tests. Les différents pointeurs d'une même cellule peuvent donc être utilisés l'un après l'autre. Il est donc impératif d'indiquer si un pointeur utilisé est le dernier de la cellule. Ceci est réalisé classiquement en faisant suivre le dernier pointeur d'une valeur particulière notée NIL.

On a représenté sur la figure 5, l'évolution du signal contenu dans le registre 12 du moyen de traitement lorsque le second moyen de codage 16 fonctionne comme un analyseur syntaxiqueà pile selon l'arbre de la figure 3.

Le signal contenu initialement dans le registre est la suite des symboles terminaux représentés sur la figure 2. La récriture de ce signal sous la forme de symboles sommés utilise une pile pour mémoriser les symboles auxquels on cherche à appliquer une règle. Sur la figure 5, l'élément "↑" représente le pointeur de pile, les éléments se trouvant à gauche étant dans la pile.

Au départ (pas 1), la pile est vide. A l'instant suivant (pas 2), la pile contient l'unique symbole "col". Aucune règle n'étant applicable, le symbole suivant "pic" est ajouté dans la pile (pas 3). Pour cet état de la pile, le test du noeud 42 est satisfait et la règle R1 (par exemple) est appliquée.

Le procédé de traitement se poursuit en testant les règles selon l'ordre défini par l'arbre de la figure 3 à chaque suite de symboles contenus dans la pile. Si aucune règle n'est applicable, un nouveau symbole du registre est introduit dans la pile (en fait, on déplace simplement le pointeur de pile) et on recherche à nouveau si une règle est applicable.

Après chaque récriture, on sort du registre les symboles sommets qui se trouvent en tête de la suite des symboles. Ainsi, dans le traitement illustré sur la figure 5,le symbole sommet "chicot" obtenu après l'application de la règle R4 au pas 4 est délivré en sortie du moyen de traitement. Il en est de même pour les symboles sommets "cuesta" et "pic-large" obtenus après l'application de la règle R6 au pas 12.

Le signal S, représenté sur la figure 2, reçu par le système de traitement est donc finalement représenté par une suite de trois symboles sommets.

L'organisation en mémoire des règles de récriture décrite en référence à la figure 4, et illustrée par la figure 3 montrant la structure en arbre correspondante, se traduit par le fait que, pour vérifier si une règle s'applique, on teste les symboles constituant l'élément gauche de la règle en partant du symbole le plus à droite et en allant vers le symbole le plus à gauche. Cette technique est employée dans les analyseurs syntaxiques, comme on l'a indiqué plus haut.

Cependant, l'invention n'est nullement limitée à une telle organisation de la mémoire et vise également les organisations de la mémoire correspondant à une recherche de la règle applicable dans laquelle on teste la suite des symboles des éléments gauches des règles de la gauche vers la droite.

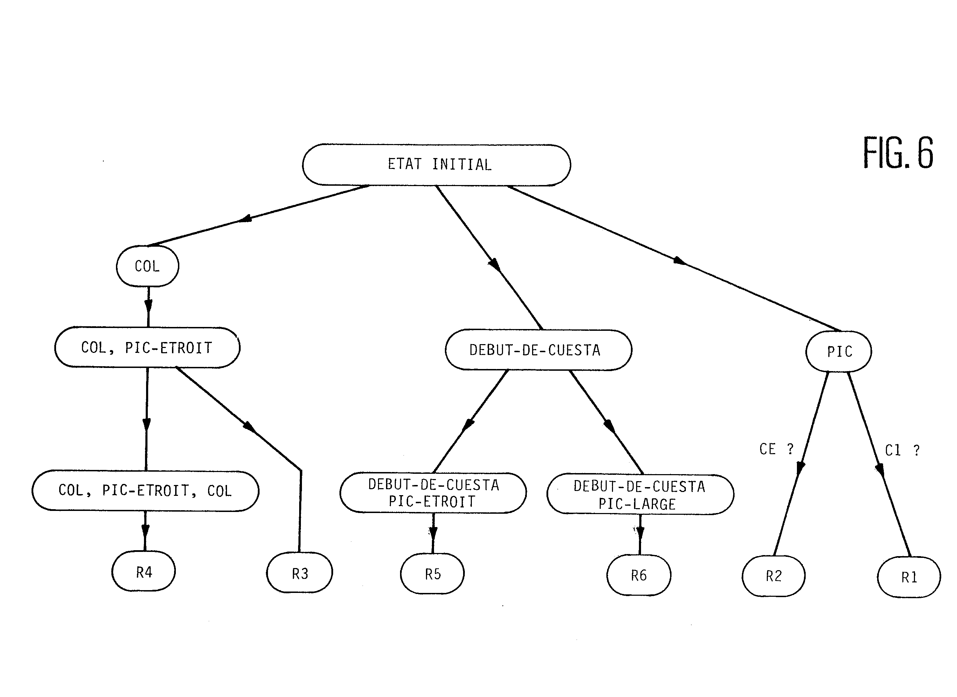

Un arbre correspondant à une telle organisation des règles en mémoire et à une telle recherche est représenté sur la figure 6.

Il n'est pas ici nécessaire d'introduire un symbole particulier "X" pour que la règle R4 soit plus prioritaire que la règle R3. Il suffit de ranger la règle R3 à droite de la règle R4 et de parcourir l'arbre de gauche à droite, et en profondeur d'abord.

Comme dans l'arbre de la figure 3, on revient en général à l'état initial après l'application d'une règle.

L'organisation en arbre des règles contenues dans la mémoire 14 permet une vitesse de traitement plus importante que dans les systèmes de traitement de l'art antérieur dans lesquels les règles sont mémorisées indépendamment les unes des autres dans une table.

Le gain en vitesse, toutes choses égales par ailleurs, est de l'ordre d'un facteur 5. Ceci permet, notamment en reconnaissance de la parole de traiter le signal de parole en temps réel.

Le système de traitement de l'invention peut être associé avantageusement à un système expert de type déclaratif. Dans ce système expert, les règles sont stockées indépendamment l'une de l'autre dans une table, et des moyens interactifs sont prévus pour permettre à un opérateur de modifier la table de règles (adjonction, suppression, modification). Ce système expert est lent et ne peut pas être utilisé pour traiter un signal de parole en temps réel.

Lorsque la base de connaissances constituée par les règles de récriture est mise au point et écrite sous forme d'arbre, elle peut être traduite sous la forme d'un programme (suite d'instructions) et constitue alors un système procédural. Celui-ci ne permet pas de modifier les règles de récriture, mais présente l'avantage d'être beaucoup plus rapide.

Une telle combinaison d'un système expert et d'un système de traitement (procédural) est décrit dans l'article "Système expert pour la reconnaissance de la parole par segmentation du signal" de S. MINAULT et al présenté aux 5èmes Journées Internationales sur les systèmes experts et leurs applications, 13-15 Mai 1985, Avignon (France).

Ce système de traitement peut avantageusement être construit conformément à l'invention. Le demandeur a réalisé un tel système de traitement de signal. Les règles de récriture sont celles qui sont indiquées dans l'article cité et elles sont mémorisées selon une structure identique à celle décrite en référence aux figures 3 et 4 du présent texte.

## Revendications

1. Système de traitement de signal caractérisé en ce qu'il comprend :

- un moyen de prétraitement (2) muni d'une entrée pour recevoir un ou plusieurs signaux monodimensionnels et d'une sortie, ledit moyen de prétraitement comprenant une mémoire (6) contenant un ensemble E de symboles $S_T$ dits terminaux, chaque symbole étant un code associé à une forme simple prédéterminée du signal, et un moyen de codage (8) pour analyser ledit signal S et délivrer sur ladite sortie dudit moyen de prétraitement une suite de symboles terminaux constituant un codage dudit signal S,

- un moyen de traitement (4) muni d'une entrée pour recevoir ladite suite de symboles terminaux, et pour délivrer une suite de symboles dits sommets correspondante, ladite suite de symboles sommets formant une représentation condensée dudit signal S, où chaque symbole sommet représente une forme complexe prédéterminée de signal égale à une suite de plusieurs formes simples, ledit moyen de traitement comprenant :

a) un registre (12) de type premier entré-premier sortie pour recevoir les symboles terminaux délivrés par le moyen de prétraitement (2),

b) une mémoire (14) contenant un ensemble de règles de récriture, chaque règle R étant de type MG → MD, où les éléments MG et MD sont des séquences de symboles comprenant des symboles sommets et des symboles terminaux, ledit ensemble de règles de récriture étant mémorisé dans des cellules de mémorisation organisées en un arbre logique comprenant : une cellule de mémorisation appelées racine, un ensemble de cellules de mémorisation appelées noeuds finaux et un ensemble de cellules de mémorisation appelées noeuds intermédiaires, ladite racine comprenant une zone pour contenir des pointeurs réalisant un chaînage avec des noeuds intermédiaires, et chaque noeud intermédiaire comprenant une zone pour contenir un symbole et une zone pour contenir des pointeurs réalisant un chaînage avec un noeud final ou au moins un autre noeud intermédiaire, lesdits chaînages formant des chemins entre la racine et chaque noeud final, chaque chemin correspondant à une séquence de symboles constituant l'élément MG d'une règle R et le noeud final situé à l'extrémité dudit chemin comprenant une zone pour contenir l'élément MD de ladite règle R, et

c) un second moyen de codage (16) pour comparer les symboles placés en tête dudit registre avec les séquences MG de chaque règle et remplacer une suite desdits symboles, identique à une séquence MG d'une règle de récriture par la séquence MD de ladite règle, ledit second moyen de codage délivrant sur ladite sortie dudit moyen de traitement chaque symbole sommet placé en tête dudit registre et répétant l'opération de remplacement des symboles en tête tant que le registre n'est pas vide, ledit second moyen de codage étant conçu pour comparer les symboles en tête dudit registre avec les éléments MG des règles rangées dans un ordre déterminé, cet ordre correspondant à parcourir ledit arbre logique en choisissant à chaque noeud intermédiaire le chemin le plus long d'abord.

2. Système de traitement selon la revendication 1, caractérisé en ce que, pour un élément MG d'une règle comprenant la suite de symboles MG(1), MG(2), ... MG(n), des noeuds intermédiaires NI(1), NI(n) formant le chemin, correspondant audit élément MG, entre l'état initial et le noeud final comprennent respectivement les symboles MG(n), MG(n1), ... MG(1).

3. Système selon la revendication 2, caractérisé en ce que chaque noeud final contenant une séquence de symbole S(1), ... S(m), $1 \leqq m$, formant un élément MD d'une règle R, qui est égale à une séquence de symboles terminant un élément $MG_1$, contient en outre un pointeur réalisant un chaînage avec le noeud intermédiaire, du chemin correspondant audit élément

MG$_1$, contenant ledit symbole S(1).

4. Système selon l'une quelconque des revendications 1 à 3 dans lequel au moins un symbole de l'ensemble des symboles terminaux comporte un attribut, caractérisé en ce que pour chaque chemin, un premier noeud intermédiaire mémorise ledit symbole et un second noeud intermédiaire mémorise la valeur dudit attribut, l'ensemble des seconds noeuds intermédiaires étant placé juste avant le noeud final.

0292351

## FIG. 1

## FIG. 2

$$E = \{COL, PIC\}$$

FIG. 3

22 — ETAT INITIAL

24 COL
26 PIC-ETROIT, COL
28 COL, PIC-ETROIT, COL
44 R4

30 X
32 PIC-ETROIT, X
34 COL, PIC-ETROIT, X
46 R7

36 DEBUT-DE-CUESTA, PIC-ETROIT, X
48 R8

38 PIC-LARGE
40 DEBUT-DE-CUESTA, PIC-LARGE
50 R6

56

42 PIC
C2 ?
R2
52

C1 ?
R1
54

0292351

# FIG.4

ADRESSES

| | | | | | |
|---|---|---|---|---|---|
| 0000 | 0001 | 0004 | 0008 | 0010 | NIL |
| 1 | COL | 0002 | NIL | | |
| 2 | PIC-ETROIT | 0003 | NIL | | |
| 3 | COL | 0013 | NIL | | |
| 4 | X | 0005 | NIL | | |
| 5 | PIC-ETROIT | 0006 | 0007 | NIL | |
| 6 | COL | 0014 | NIL | | |
| 7 | DDC* | 0015 | NIL | | |
| 8 | PIC-LARGE | 0009 | NIL | | |
| 9 | DDC* | 0016 | NIL | | |
| 10 | PIC | 0011 | 0012 | NIL | |
| 11 | 1 > L1 | 0017 | NIL | | |
| 12 | 1 < L1 | 0018 | NIL | | |
| 13 | R | CHICOT | 0001 | | |
| 14 | R | DDC* | 0001 | | |
| 15 | R | DDC* | 0001 | | |
| 16 | R | DDC* | PIC-LARGE | 0001 | |
| 17 | R | PIC-LARGE | 0008 | | |
| 18 | R | PIC-ETROIT | 0001 | | |

DDC* = DEBUT-DE-CUESTA

# FIG. 5

0292351

| PAS | CONTENU DU REGISTRE | REGLE APPLICABLE |
|---|---|---|
| 1 | ↑ COL  PIC  COL  PIC ·· PIC  PIC  PIC | |
| 2 | COL ↑ PIC  COL  PIC  PIC  PIC  PIC | |
| 3 | COL  PIC ↑ COL  PIC  PIC  PIC  PIC | R1 |
| 4 | COL  PIC ETROIT  COL ↑ PIC  PIC  PIC  PIC | R4 |
| 5 | [CHICOT]  COL  PIC ↑ PIC  PIC  PIC | R1 |
| 6 | COL  PIC ETROIT  PIC ↑ PIC  PIC | R7 |
| 7 | DEBUT-DE CUESTA  PIC ↑ PIC  PIC | R1 |
| 8 | DEBUT-DE CUESTA  PIC ETROIT  PIC ↑ PIC | R8 |
| 9 | DEBUT-DE CUESTA  PIC ↑ PIC | R1 |
| 10 | DEBUT-DE CUESTA  PIC ETROIT  PIC ↑ | R8 |
| 11 | DEBUT-DE CUESTA  PIC ↑ | R2 |
| 12 | DEBUT-DE CUESTA  PIC LARGE ↑ | R6 |
| 13 | [CUESTA]  [PIC LARGE] ↑ | |

FIG. 6

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | IEEE TRANSACTIONS ON ACOUSTICS, SPEECH, AND SIGNAL PROCESSING, vol. ASSP-24, no. 5, octobre 1976, pages 365-378, New York, US; R. DE MORI et al.: "Automatic detection and description of syllabic features in continuous speech" * Pages 365-370; page 378 "Conclusions" * | 1 | G 06 F 15/74 |
| A | IEEE PROCEEDINGS OF THE CONFERENCE ON COMPUTER GRAPHICS, PATTERN RECOGNITION AND DATA STRUCTURE, Long Beach, 14-16 mai 1975, pages 135-138; S.C. LEE: "Syntactic pattern recognition of EEG spikes" * Pages 135-136; figures 1-3 * | 1 | |
| A | IEEE TRANSACTIONS ON BIOMEDICAL ENGINEERING, vol. BME-26, no. 3, mars 1979, pages 125-136, IEEE, New York, US; G. BELFORTE et al.: "A contribution to the automatic processing of electrocardiograms using syntactic methods" * Page 125, colonne 2, paragraphe 5 - page 132, colonne 1, paragraphe 5 * | 1 | |
| A | PROCEEDINGS IEEE COMPUTER SOCIETY CONFERENCE ON PATTERN RECOGNITION AND IMAGE PROCESSING, Las Vegas, Nevada, 14-17 juin 1982, pages 18-27, IEEE, New York, US; K.S. FU: "Attributed grammars for pattern recognition - a general (syntactic-semantic) approach" | | |

**DOMAINES TECHNIQUES RECHERCHES (Int. Cl.4)**

G 06 F 15/20
G 06 F 15/74
G 06 K 9/68

Le présent rapport a été établi pour toutes les revendications

| Lieu de la recherche | Date d'achèvement de la recherche | Examinateur |
|---|---|---|
| LA HAYE | 22-08-1988 | CHUGG D.J. |

EPO FORM 1503 03.82 (P0402)